# EUROPÄISCHE PATENTSCHRIFT

(11) **EP 1 146 846 B1**
(45) Veröffentlichungstag und Bekanntmachung des Hinweises auf die Patenterteilung: **14.12.2005**
(21) Anmeldenummer: 00969498.5
(22) Anmeldetag: 13.10.2000
(51) Int. Cl.: A61K 7/06

(54) **KLARES HAARBEHANDLUNGSMITTEL**
CLEAR HAIR-TREATMENT AGENT
PRODUIT TRANSPARENT DE TRAITEMENT CAPILLAIRE

(30) Priorität: 19.10.1999 DE 19950219
(43) Veröffentlichungstag der Anmeldung: 24.10.2001
(73) Patentinhaber: Wella Aktiengesellschaft, 64274 Darmstadt (DE)
(72) Erfinder: SCHMENGER, Jürgen, 64331 Weiterstadt (DE); ABELS, Wilhelm, Simi Valley, CA 93065 (US); JAHEDSHOAR, Mehrdad, Calabasas, CA 91302 (US)
(86) Internationale Anmeldenummer: PCT/EP2000/010081
(87) Internationale Veröffentlichungsnummer: WO 2001/028505

(56) Entgegenhaltungen:
- EP-A- 0 179 347
- EP-A- 0 870 491
- WO-A-95/09599
- WO-A-97/46211

## Beschreibung

Gegenstand der Erfindung ist ein Haarbehandlungsmittel, welches insbesondere als Leave-in Haarkur oder als Haarspülung anwendbar ist, vorzugsweise in Form eines optisch klaren Produktes vorliegt und einen Gehalt an hydrophilen Silikonen und bestimmten Assoziatiwerdickern sowie gegebenenfalls kationaktiven, haarpflegenden Stoffen aufweist.

Übliche haarkonditionierende Präparate wie Rinse-off Kuren oder Leave-on Treatments sind in der Regel auf der Basis von wässrigen Emulsionen formuliert. Wesentliche Inhaltsstoffe sind kationaktive Substanzen wie z.B. kationische Tenside, hydrophobe Substanzen wie z.B. Fettalkohole und andere Ölkomponenten, Emulgatoren, sowie weitere spezifische Wirk- und Duftstoffe. Die wichtigsten Bestandteile sind dabei kationische Tenside, Fettalkohole und Emulgatoren. Einen Überblick über den prinzipiellen Aufbau von Kurspülungen und Haarkuren gibt Schrader, 'Grundlagen und Rezepturen der Kosmetika', 2. Auflage, 1989, Seiten 728 bis 737. Hauptaufgaben der Konditioniermittel sind die Verbesserung der Frisierbarkeit, der Kämmbarkeit, des Glanzes und des Griffs des behandelten Haares. Die behandelten Haare fühlen sich häufig etwas schwerer und belasteter an, was nicht immer erwünscht ist. Die herkömmlichen O/W-Haarkuremulsionen sind darüberhinaus normalerweise milchig-weiß und undurchsichtig. Wünschenswert sind Produkte, welche in einer optisch ansprechenderen Form vorliegen und klar, durchsichtig oder zumindest durchscheinend sind. Verschiedene Formen klarer Haarkurmittel sind zwar bekannt und beispielsweise beschrieben in E. Flick, "Cosmetic and Toiletry Formulations", Second Edition Volume 2, Seiten 373 ff. Diese klaren Haarkurmittel sind auf Basis von verdickend wirkenden Polymeren wie z.B. Cellulosederivaten (Handelsnamen Natrosol®, Methocel®), hochmolekularen Chitosanderivaten (Handelsname Kytamer® PC), komplexen Polysacchariden (Handelsnamen Karaya Gum, Tragant, Jaguar® Typen, Keltrol® Typen) und Acrylsäurepolymerisaten hergestellt. Alle diese beschriebenen klaren Haarkurmittel haben den großen Nachteil, daß die Pflegewirkung so schwach ist, daß sie bei weitem nicht diejenige eines klassischen Haarkurmittels auf Basis von Mischungen von Fettalkoholen und quaternären Tensiden erreicht. Diese, nach dem Stand der Technik bekannten klaren Haarkurmittel verkaufen sich deshalb auf dem Markt deutlich schlechter als die Standardkuren.

Es bestand somit die Aufgabe, ein Mittel zur Verfügung zu stellen, welches die typischen, an ein Haarkonditioniermittel zu stellenden Anforderungen hinsichtlich Haarkonditionierung erfüllt und gleichzeitig in einer optisch ansprechenden Form vorliegt und dem Haar möglichst einen weniger schweren und belasteten Eindruck verleiht wie nach einer Behandlung mit einem herkömmlichen Kurmittel.

Es wurde nun gefunden, daß die Aufgabe gelöst wird durch ein Haarbehandlungsmittel der nachfolgend beschriebenen Zusammensetzung. Gegenstand der Erfindung ist ein Haarbehandlungsmittel mit einem Gehalt an
(A) mindestens einer haarpflegenden Silikonverbindung, welche mindestens eine hydrophile Gruppe enthält und
(B) mindestens einem nichtionischen, amphiphilen Assoziativverdicker, ausgewählt aus hydrophob modifizierten Aminoplast/Polyether Copolymeren
in einer geeigneten kosmetischen Basis.
Vorzugsweise enthält das erfindungsgemäße Mittel zusätzlich mindestens einen haarpflegenden Stoff (C), welcher mindestens eine kationaktive Gruppe enthält.

Das Mittel erfüllt die an ein Haarkonditioniermittel hinsichtlich Konditionierwirkung zu stellenden Anforderungen in bester Weise. Das Haar ist nach der Behandlung sowohl im feuchten als auch im trockenen Zustand merkbar glatter und die Nasskämmbarkeit ist merkbar verbessert. Überraschenderweise wurde gefunden, daß das Verdickungsmittel erlaubt, kationische Stoffe und die genannten Silikonverbindungen einzuarbeiten, ohne dabei negative Begleiteigenschaften des Verdickungsmittels zu fühlen. Die fachlichen Eigenschaften des erfindungsgemäßen Mittels übertreffen sogar noch die Eigenschaften einer konventionellen Haarkur auf Basis einer wäßrigen Emulsion von Fettalkoholen und quaternären Tensiden. Salon Tests im Halbseitenvergleich bestätigen dem erfindungsgemäßen Mittel eine bessere Kämmbarkeit und ein natürlicheres Anfühlen der Haare. Der meistens zu beobachtende negative stumpfe Griff der Haare von Fettalkohol/Kationtensid Mischungen ist mit dem erfindungsgemäßen Mittel praktisch eliminiert. Die Haare fühlen sich leichter und unbelasteter an. Darüberhinaus ermöglicht die erfindungsgemäße Kombination, daß das Mittel in einer optisch ansprechenden, klaren Formulierung konfektioniert werden kann, was wiederum die vorteilhafte Abpackung in einem transparenten Behälter, beispielsweise aus Glas oder durchsichtigem Kunststoff, z.B. Polyethylen, Polypropylen oder Polyethylenterephtalat ermöglicht.

Der kationaktive Stoff (C) ist in dem erfindungsgemäßen Mittel vorzugsweise in einer Menge von 0,01 bis 10, besonders bevorzugt von 0,1 bis 5 Gewichtsprozent enthalten und unterscheidet sich von den Silikonverbindungen (A), indem er keine Silikoneinheiten enthält. Die Silikonverbindung (A) ist in einer Menge von vorzugsweise 0,01 bis 10 Gew.%, besonders bevorzugt von 0,1 bis 5 Gew.% und der Assoziatiwerdicker (B) in einer Menge von vorzugsweise 0,1 bis 5 Gew.%; besonders vevorzugt von 0,1 bis 2 Gew.% enthalten.

Der kationaktive Stoff (C) ist eine Substanz, die auf Grund von kationischen oder kationisierbaren Gruppen, insbesondere primären, sekundären, tertiären oder quaternären Amingruppen eine Substantivität zu menschlichem Haar aufweist. Geeignete kationaktive Stoffe sind ausgewählt aus kationischen Tensiden, betainischen Tensiden, amphoteren Tensiden, kationaktiven Polymeren mit kationischen oder kationisierbaren Gruppen, kationisch derivatisierten Proteinen, kationisch derivatisierten Proteinhydrolysaten und Betain.

Geeignete kationaktive Tenside sind Tenside, welche eine quaternäre Ammoniumgruppe enthalten. Dabei kann es sich um kationische oder um amphotere, betainische Tenside handeln. Besonders bevorzugt als kationaktiver Stoff (C) sind kationische Tenside. Geeignete kationische Tenside enthalten Aminogruppen oder quaternisierte hydrophile Ammoniumgruppen, welche in Lösung eine positive Ladung tragen und durch die allgemeine Formel (I) dargestellt werden können,

N⁽⁺⁾R¹R²R³R⁴ X⁽⁻⁾ (I)

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁽⁻⁾ ein Anion darstellt, beispielsweise ein Halogen, Acetat, Phosphat, Nitrat oder Alkylsulfat, vorzugsweise ein Chlorid. Die aliphatischen Gruppen können zusätzlich zu den Kohlenstoffatomen und den Wasserstoffatomen auch Querverbindungen oder andere Gruppen wie beispielsweise Hydroxygruppen oder weitere Aminogruppen enthalten.

Beispiele für geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder-bromide, die Dialkyldimethylammoniumchloride oder-bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt sind Cetyltrimethylammoniumchlorid, das beispielsweise in Form einer 26prozentigen wässrigen Lösung unter der Handelsbezeichnung Dehyquart® A von der Firma Henkel KGaA, Düsseldorf/Deutschland und unter der Handelsbezeichnung Genamin® CTAC von der Firma Hoechst AG, Frankfurt/Deutschland sowie in Form einer 50prozentigen Lösung in Isopropanol unter der Handelsbezeichnung Arquad® 16-50 von der Firma Akzo Chemicals GmbH, Düren/Deutschland vertrieben wird.

Geeignete amphotere Tenside sind Derivate aliphatischer quatemärer Ammonium-, Phosphonium- und Sulfoniumverbindungen der Formel (II) 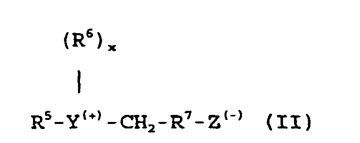 wobei R5 eine geradkettige oder verzweigtkettige Alkyl-, Alkenyl- oder Hydroxyalkylgruppe mit 8 bis 18 Kohlenstoffatomen und 0 bis etwa 10 Ethylenoxideinheiten und 0 bis 1 Glycerineinheiten darstellt; Y eine N-, P- oder S-haltige Gruppe ist; R6 eine Alkyl- oder Monohydroxyalkylgruppe mit 1 bis 3 Kohlenstoffatomen ist; x gleich 1 ist, falls Y ein Schwefelatom ist und x gleich 2 ist, wenn Y ein Stickstoffatom oder ein Phosphoratom ist; R7 eine Alkylen- oder Hydroxyalkylengruppe mit 1 bis 4 Kohlenstoffatomen ist und Z⁽⁻⁾ eine Carboxylat-, Sulfat-, Phosphonat- oder Phosphatgruppe darstellt.

Andere amphotere Tenside wie Betaine sind ebenso geeignet für das erfindungsgemäße Haarbehandlungsmittel. Beispiele für Betaine umfassen C8- bis C18-Alkylbetaine wie Cocodimethylcarboxymethylbetain, Lauryldimethylcarboxymethylbetain, Lauryldimethylalphacarboxyethylbetain, Cetyldimethylcarboxymethylbetain, Oleyldimethylgammacarboxypropylbetain und Lauryl-bis(2-hydroxypropyl)alphacarboxyethylbetain; C8- bis C18-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die C8- bis C18-Alkyldimethylammoniumacetate, die C8- bis C18-Alkyldimethylcarbonylmethylammoniumsalze sowie die C8- bis C18-Fettsäurealkylamidobetaine wie beispielsweise das Kokosfettsäureamidopropylbetain, welches beispielsweise in Form einer 30%igen wäßrigen Lösung unter der Handelsbezeichnung Tego® Betain L7 von der Firma Goldschmidt AG vertrieben wird und das N-Kokosfettsäureamidoethyl-N-[2-(carboxymethoxy)ethyl]-glycerin (CTFA-Name: Cocoamphocarboxyglycinate), welches z.B. in Form einer 50%igen wäßrigen Lösung unter der Handelsbezeichnung Miranol® C2M von der Firma Miranol Chemical Co. Inc. vertrieben wird.

Bei den geeigneten kationaktiven Polymeren handelt es sich vorzugsweise um haarfestigende oder um haarkonditionierende Polymere. Geeignete Polymere der Komponente (C) enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quatemisiertes Vinylpyrrolidon/Dimethylaminoethylmethacrylat Copolymer (Polyquaternium-11).

Von den kationischen Polymeren, die in dem erfindungsgemäßen Mittel enhalten sein können, ist zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und von denen das Gafquat® 734 besonders bevorzugt ist, geeignet. Weitere kationische Polymere sind beispielsweise das von der Firma BASF, Deutschland unter dem Handelsnamen LUVIQUAT® HM 550 vertriebene Copolymer aus Polyvinylpyrrolidon und Imidazoliminmethochlorid, das von der Firma Calgon/USA unter dem Handelsnamen Merquat® Plus 3300 vertriebene Terpolymer aus Dimethyldiallylammoniumchlorid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam und das von der Firma Gaf unter dem Handelsnamen Gafquat® HS 100 vertriebene Vinylpyrrolidon/Methacrylamidopropyltrimethylammoniumchlorid Copolymer.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel (III)

G-O-B-N⁺R^{a}R^{b}R^{c} X⁻ (III)

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R^{a}, R^{b} und R^{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R^{a}, R^{b} und R^{c} vorzugsweise maximal 20 ist;
X⁽⁻⁾ ist ein übliches Gegenanion, hat die gleiche Bedeutung wie bei Formel (I) und ist vorzugsweise Chlorid. Eine kationische Cellulose wird unter der Bezeichnung Polymer JR von Amerchol vertrieben und hat die INCI-Bezeichnung Polyquaternium-10. Eine weitere kationische Cellulose trägt die INCI-Bezeichnung Polyquatemium-24 und wird unter dem Handelsnamen Polymer LM-200 von Amerchol vertrieben. Ein geeignetes kationisches Guarderivat wird unter der Handelsbezeichnung Jaguar® R vertrieben und hat die INCI-Bezeichnung Guar Hydroxypropyltrimonium Chloride.

Besonders bevorzugte kationaktive Stoffe sind Chitosan, Chitosansalze und Chitosan-Derivate. Bei den erfindungsgemäß einzusetzenden Chitosanen handelt es sich um vollständig oder partiell deacetylierte Chitine. Zur Herstellung von Chitosan geht man vorzugsweise von dem in den Schalenresten von Krustentieren enthaltenem Chitin aus, welches als billiger und natürlicher Rohstoff in großen Mengen zur Verfügung steht. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%.

Ein geeignetes Chitosan wird beispielsweise von der Firma Kyowa Oil&Fat, Japan, unter dem Handelsnamen Flonac® vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches beispielsweise unter der Bezeichnung Kytamer PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosan-derivate kommen quaternierte, alkylierte oder hydroxyalkylierte Derivate, beispielsweise Hydroxyethyl- oder Hydroxybutylchitosan in Betracht.

Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie z.B. Ameisensäure, Weinsäure, Äpfelsäure, Milchsäure, Zitronensäure, Pyrrolidoncarbonsäure, Salzsäure u.a., von denen die Pyrrolidoncarbonsäure besonders bevorzugt ist.

Weitere geeignete kationaktive, haarpflegende Verbindungen sind kationisch modifizierte Proteinderivate oder kationisch modifizierte Proteinhydrolysate und sind beispielsweise bekannt unter den INCI-Bezeichnungen Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein oder Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, Hydroxypropyltrimonium Hydrolyzed Vegetable Protein.

Geeignete kationisch derivatisierte Proteinhydrolysate sind Substanzmischungen, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können. Proteine, die als Ausgangsstoffe für die Proteinhydrolysate dienen, können sowohl pflanzlicher als auch tierischer Herkunft sein. Übliche Ausgangsstoffe sind beispielsweise Keratin, Collagen, Elastin, Sojaprotein, Reisprotein, Milchprotein, Weizenprotein, Seidenprotein oder Mandelprotein. Durch die Hydrolyse entstehen Stoffmischungen mit Molmassen im Bereich von ca. 100 bis ca. 50.000. Übliche mittlere Molmassen liegen im Bereich von etwa 500 bis etwa 1000. Vorteilhafterweise enthalten die kationisch derivatisierten Proteinhydrolysate eine oder zwei lange C8- bis C22-Alkylketten und entsprechend zwei oder eine kurze C1- bis C4-Alkylketten. Verbindungen, die eine lange Alkylkette enthalten, sind bevorzugt.

Die hydrophilen Gruppen der erfindungsgemäß einzusetzenden haarpflegenden Silikonverbindungen (A) sind vorzugsweise ausgewählt aus Hydroxylgruppen, primären, sekundären oder tertiären Aminogruppen, quaternären Ammoniumgruppen, Alkylenoxidgruppen, betainischen Gruppen und Thiosulfatgruppen.

Geeignet und besonders bevorzugt sind kationaktive Silikonverbindungen. Diese sind mit kationischen oder kationisierbaren Gruppen substituiert. Geeignete kationaktive Silikonvberbindungen weisen entweder mindestens eine Aminogruppe oder mindestens eine Ammoniumgruppe auf. Geeignete Silikonpolymere mit Aminogruppen sind unter der INCl-Bezeichnung Amodimethicone bekannt. Hierbei handelt es sich um Polydimethylsiloxane mit Aminoalkylgruppen. Die Aminoalkylgruppen können seiten- oder endständig sein. Geeignete Aminosilikone sind solche der allgemeinen Formel (IV)

R⁸R⁹R¹⁰Si-(OSiR¹¹R¹²)x-(OSiR¹³Q)y-OSiR¹⁴R¹⁵R¹⁶ (IV)

R⁸, R⁹, R¹⁴ und R¹⁵ sind unabhängig voneinander gleich oder verschieden und bedeuten C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl; R¹⁰ und R¹⁶ sind unabhängig voneinander gleich oder verschieden und bedeuten -(CH₂)ₐ-NH₂ mit a gleich 1 bis 6, C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;
R¹¹, R¹² und R¹³ sind unabhängig voneinander gleich oder verschieden und bedeuten Wasserstoff, C1- bis C20-Kohlenwasserstoff, welcher O- und N-Atome enthalten kann, vorzugsweise C1- bis C10-Alkyl oder Phenyl, besonders bevorzugt C1- bis C4-Alkyl, insbesondere Methyl;
Q bedeutet -A-NR¹⁷R¹⁸, oder -A-N⁺R¹⁷R¹⁸R¹⁹ wobei A für eine divalente C1- bis C20-Alkylenverbindungsgruppe steht, welche auch O- und N-Atome sowie OH-Gruppen enthalten kann, und R¹⁷, R¹⁸ und R¹⁹ unabhängig voneinander gleich oder verschieden sind und Wasserstoff, C1- bis C22-Kohlenwasserstoff, vorzugsweise C1- bis C-4-Alkyl oder Phenyl bedeuten. Bevorzugte Reste für Q sind -(CH₂)₃-NH₂, -(CH₂)₃NHCH₂CH₂NH₂, -(CH₂)₃OCH₂CHOHCH₂NH₂ und -(CH₂)₃N(CH₂CH₂OH)₂, -(CH₂)₃-NH₃⁺ und -(CH₂)₃OCH₂CHOHCH₂N⁺(CH₃)₂R²⁰, wobei R²⁰ ein C1- bis C22-Alkylrest ist, der auch OH-Gruppen aufweisen kann; x bedeutet eine Zahl zwischen 1 und 10.000, vorzugsweise zwischen 1 und 1.000; y bedeutet eine Zahl zwischen 1 und 500, vorzugsweise zwischen 1 und 50.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 0,1 bis 0,5.

Geeignete Silikonpolymere mit zwei endständigen quaternären Ammoniumgruppen sind unter der INCI-Bezeichnung Quaternium-80 bekannt. Hierbei handelt es sich um Dimethylsiloxane mit zwei endständigen Aminoalkylgruppen. Geeignete quaternäre Aminosilikone sind solche der allgemeinen Formel (V)

R²¹R²²R²³N⁺-A-SiR⁸R⁹-(OSiR¹¹R¹²)ₙ-OSiR⁸R⁹-A-N⁺R²¹R²²R²³ 2X⁻ (V)

A hat die gleiche Bedeutung wie oben bei Formel (IV) angegeben und ist vorzugsweise -(CH₂)₃OCH₂CHOHCH₂N⁺(CH₃)₂R²⁰, wobei R²⁰ ein C1- bis C22-Alkylrest ist, der auch OH-Gruppen aufweisen kann;
R⁸, R⁹, R¹¹ und R¹² haben die gleiche Bedeutung wie oben bei Formel (IV) angegeben und sind vorzugsweise Methyl;
R²¹, R²², und R²³ bedeuten unabhängig voneinander C1-bis C22-Alkylreste, welche Hydroxygruppen enthalten können und wobei vorzugsweise mindestens einer der Reste mindestens 10 C-Atome aufweist und die übrigen Reste 1 bis 4 C-Atome aufweisen; n ist eine Zahl von 0 bis 200, vorzugsweise von 10 bis 100. Derartige diquaternäre Polydimethylsiloxane werden von der Firma GOLDSCHMIDT/Deutschland unter den Handelsnamen Abil® Quat 3270, 3272 und 3274 vertrieben.

Geeignete Silikone mit Alkylenoxidgruppen sind Polydimethylsiloxane mit polyoxyalkylierten Substituenten, insbesondere Silikone, die mit Polypropylenoxid, Polyethylenoxid oder deren Gemisch modifiziert sind. Die Alkylenoxidgruppen können dabei seitenständig oder endständig sein oder es kann sich um Polydimethylsiloxan/Polyalkylenoxid Blockcopolymere handeln. Die mit Alkylenoxiden modifizierten Siloxane werden auch als Dimethylsiloxanglykolcopolymere oder als Dimethicon Copolyole bezeichnet. Geeignete Silikone mit Hydroxylgruppen sind Dimethiconole. Hierbei handelt es sich um Polydimethylsiloxane mit Hydroxylendgruppen. Geeignete Silikone mit Thiosulfatgruppen sind bekannt unter der INCI-Bezeichnung Dimethicone/Sodium PG-Propyldimethicone Thiosulfate Copolymer.

Der nichtionische, amphiphile Assoziatiwerdicker (B) ist ein Polymer, welches sowohl hydrophile als auch hydrophobe Gruppen enthält. Assoziatiwerdicker sind wasserlösliche Polymere und haben tensidartige hydrophobe Bestandteile, welche in der Lage sind, sich in einem hydrophilen, insbesondere wäßrigen Medium sowohl mit sich selbst als auch mit anderen hydrophoben Stoffen zu assoziieren, d.h. in Wechselwirkung zu treten. Durch das daraus resultierende assoziative Netzwerk wird das Medium verdickt oder geliert. Typischerweise werden Assoziativverdicker hergestellt durch Polymerisation von Polyethylenoxid-Prepolymeren und mindestens zweifach funktionellen, polykondensierbaren Stoffen wie z.B. Isocyanaten, wobei Mono- oder Diole mit großen Aryl-, Alkyl- oder Aryl/Alkyl-Gruppen eingebaut werden, um die hydrophobe Modifikation bereitzustellen. Bevorzugte Assoziatiwerdicker sind daher hydrophob modifizierte Polyalkylenglykole. Hierbei wird der hydrophile Bestandteil durch Polyoxyalkyleneinheiten, vorzugwseise Polyoxyethylen- aber auch Polyoxypropyleneinheiten oder deren Gemisch gebildet. Der hydrophobe Bestandteil wird vorzugsweise aus Kohlenwasserstoffgruppen, z.B langkettigen Alkylgruppen, Alkylaryl- oder Arylalkyl-gruppen gebildet.

Die Assoziativverdicker sind hydrophob modifizierte Aminoplast-Polyether Copolymere. Bezüglich deren Struktur und Herstellung wird auf die WO 96/40815 verwiesen. In der WO 96/40815 werden wasserdispergierbare oder wasserlösliche Copolymere beschrieben, welche die Reaktionsprodukte sind einer säurekatalysierten Polykondensation von mindestens zweifach funktionellen Aminoplastmonomeren und mindestens zweifach funktionellen Alkylenpolyethem sowie einfach funktionellen Verbindungen mit hydrophoben Gruppen. Geeignete Aminoplaste sind der Figur 1 der WO 96/40815 zu entnehmen. Besonders bevorzugt sind die Glycolurilderivate der Formel X der WO 96/40815. Geeignete Alkylenpolyether sind der Figur 2 der WO 96/40815 zu entnehmen. Bevorzugte Alkylenpolyether sind Polyethylenoxiddiole. Diese können einen Ethoxylierungsgrad von 20 bis 500, vorzugsweise 50 bis 350, besonders bevorzugt von 100 bis 250 haben. Geeignete einfach funktionelle Verbindungen mit hydrophoben Gruppen sind diejenigen der Formel XIV der WO 96/40815.
Erfindungsgemäß geeignete Assoziativverdicker sind ausgewählt aus Polymeren der allgemeinen Formel (VI) 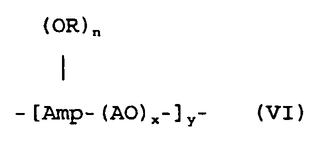 wobei Amp ein Aminoplastmonomer oder den Rest eines Aminoplastoligomers oder -polymers bedeutet,
AO für eine Alkylenoxidgruppe steht, R für Wasserstoff, C1-C4-Alkyl oder C1-C4-Acyl steht und x und y Zahlen größer 1 sind.

Besonders bevorzugt sind die Reaktionsprodukte der säurekatalysierten Polykondensation von (a) Glykolurilen der allgemeinen Formel (VII), 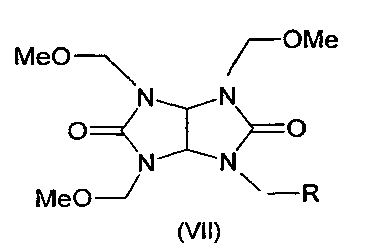 wobei R für H oder vorzugsweise für OMe steht mit (b) Polyethylenoxiddiolen eines Ethoxylierungsgrades von 20 bis 500, vorzugsweise 50 bis 350, besonders bevorzugt von 100 bis 250 sowie (c) eines gegebenenfalls ethoxylierten hydrophoben Alkohols, Alkylphenols, Thiols, Carboxamids, Carbamats oder einer hydrophoben Carbonsäure, wie sie auf den Seiten 17 bis 19 der WO 96/40815 beschrieben sind. Besonders bevorzugtes Glykoluril ist 1,3,4,6-Tetramethoxymethylglycoluril.

Geeignete Assoziativverdicker sind solche mit den INCI-Bezeichnungen Polyether-1, PEG-180/Octoxynol-40/TMMG Copolymer und PEG-180/Laureth-50/TMMG Copolymer und werden von der Firma Süd-Chemie vertrieben unter den Handelsbezeichnungen Pure-Thix® HH, L und M.

Das erfindungsgemäße Mittel wird bevorzugt in einem wäßrigen oder in einem wäßrig-alkoholischen Milieu konfektioniert und zeichnet sich besonders durch seine Klarheit und Transparenz aus. Daher wird das Mittel vorteilhafterweise auch in eine optisch ansprechende Verpackung aus durchsichtigem oder durchscheinendem Material abgefüllt. Als Verpackungsmaterial kommen insbesondere Glas und und durchsichtige oder durchscheinende Kunststoffe wie z.B. Polyethylenterephtalat in Betracht. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Der Wassergehalt beträgt vorzugsweise von 40 bis 95, besonders bevorzugt von 60 bis 90 Gewichtsprozent. Der Alkoholgehalt beträgt vorzugsweise von 1 bis 30, besonders bevorzugt von 5 bis 20 Gewichtsprozent. Weitere, besonders bevorzugte wasserlösliche Lösungs- bzw. Feuchthaltemittel sind mehrwertige Alkohole, insbesondere solche mit 2 bis 4 Kohlenstoffatomen wie beispielsweise Glycerin, Ethylenglykol oder Propylenglykol in einer Menge von 0,1 bis 10 Gew.%, vorzugsweise von 0,5 bis 5 Gew.%. Besonders bevorzugt sind rein wäßrige Formulierungen.

In einer bevorzugten Ausführungsform enthält das erfindungsgemäße Mittel zusätzlich mindestens ein nichtionisches Tensid. Geeignete nichtionische Tenside sind beispielsweise die im "International Cosmetic Ingredient Dictionary and Handbook", 7. Auflage, Band 2 im Abschnitt "Surfactants - Emulsifying Agents" aufgeführten nichtionischen Emulgatoren. Geeignete nichtionische Tenside sind vorzugsweise ausgewählt aus ethoxylierten Fettsäuren mit 10 bis 26 Kohlenstoffatomen, ethoxylierten ein- oder mehrwertigen Alkoholen mit 1 bis 6 Kohlenstoffatomen, ethoxylierten Fettalkoholen mit 10 bis 26 Kohlenstoffatomen, ethoxylietem hydriertem oder nicht hydriertem Rizinusöl, Alkylpolyglucosiden, Glyceridalkoxylaten, Fettsäureglyceridpolyalkylenglykolethern oder Fettsäurepartialglyceridpolyalkylenglykolethern mit jeweils weniger als 30 Alkylenglykoleinheiten wie z.B. Polyethylenglykol-(7)-glycerylcocoat, Polyglykolamiden, Fettsäurezuckerestern, ethoxylierten Fettsäurezuckerestem und Partialglyceriden. Der Ethoxylierungsgrad von ethoxylierten Tensiden beträgt üblicherweise von 1 bis 400, vorzugsweise 2 bis 200, besonders bevorzugt 3 bis 25.

In einer bevorzugten Ausführungsform sind in dem erfindungsgemäßen Mittel nur solche Tenside und Emulgatoren enthalten, welche wasserlöslich sind, d.h. solche Tenside, die bei einem Gehalt von 1 Gew.% in Wasser bei 20°C klar löslich sind.

Bevorzugte nichtionische Tenside sind insbesondere Fettalkoholethoxylate. Geeignet sind beispielsweise Alkohole mit 10 bis 18, vorzugsweise 10 bis 16 C-Atomen und einem Ethoxylierungsgrad von vorzugsweise 2 bis 200, besonders bevorzugt von 3 bis 25. Die zusätzlichen nichtionischen Tenside werden in einer Menge von vorzugsweise 0,01 bis 5 Gew.% eingesetzt.

In einer weiteren bevorzugten Ausführungsform enthält das erfindungsgemäße Mittel zusätzlich mindestens ein filmbildendes, haarfestigendes, synthetisches oder natürliches Polymer. Dieses zusätzliche Polymer kann nichtionischen, anionischen oder amphoteren Charakter haben und wird bevorzugt in einer Menge von 0,5 bis 10 Gew. % eingesetzt. Unter filmbildenden, haarfestigenden Polymeren werden solche Polymere verstanden, welche in 0.1 bis 5 %iger wäßriger, alkoholischer oder wäßrig-alkoholischer Lösung angewandt in der Lage sind, auf dem Haar einen Polymerfilm abzuscheiden und auf diese Weise das Haar zu festigen.

Das erfindungsgemäße Mittel kann darüber hinaus die für Haarbehandlungsmittel üblichen Zusatzbestandteile enthalten, z.B. nicht festigende nichtionische Polymere, nicht festigende anionische Polymere und nicht festigende natürliche Polymere sowie deren Kombination in einer Menge von vorzugsweise 0,01 bis 10 Gew. %; Parfümöle in einer Menge von vorzugsweise 0,01 bis 5 Gew. %; Netzmittel oder Emulgatoren aus den Klassen der anionischen, kationischen, amphoteren oder nichtionogenen oberflächenaktiven Substanzen in einer Menge von vorzugsweise 0.01 bis 10 Gew. %; Feuchthaltemittel; Konservierungsmittel, bakterizide und fungizide Wirkstoffe wie z.B. 2,4,4-Trichlor-2-hydroxydiphenylether, Parabene oder Methylchlorisothiazolinon, in einer Menge von 0,01 bis 1,0 Gew.%; Puffersubstanzen, wie z.B. Natriumcitrat oder Natriumphosphat, in einer Menge von 0,1 bis 1,0 Gew.%; Anfärbestoffe, wie z.B. Fluorescein Natriumsalz, in einer Menge von etwa 0,1 bis 1,0 Gew.%; Pflegestoffe, wie z.B. Pflanzen- und Kräuterextrakte, Protein- und Seidenhydrolysate, Lanolinderivate, in einer Menge von 0,1 bis 5 Gew.%; Lichtschutzmittel, Antioxidantien, Radikalfänger, Antischuppenwirkstoffe, Fettalkohole, Glanzgeber, Vitamine und rückfettende Agenzien in einer Menge von 0,01 bis 10 Gew.%.

Das erfindungsgemäße Mittel kann in einem pH-Bereich von 2,0 bis 9,5 vorliegen. Besonders bevorzugt sind schwach saure pH-Werte im Bereich zwischen 4,5 und kleiner 7, besonders bevorzugt von 5,5 bis 6,5. Liegt das erfindungsgemäße Mittel im sauren Bereich vor, so kann es organische oder anorganische Säuren enthalten wie beispielsweise Ameisensäure, Weinsäure, Äpfelsäure, Maleinsäure, Fumarsäure, Glyoxylsäure, Pyrrolidoncarbonsäure, Zitronensäure, Milchsäure, Schwefelsäure, Essigsäure, Salzsäure, Phosphorsäure u.a..

Das Mittel kann als Lotion, verdickte Lotion, Flüssiggel oder als hochviskoses Gel vorliegen. Vorzugsweise liegt es in mittelviskoser Form vor, d.h. es hat vorzugsweise die Konsistenz einer verdickten Lotion oder eines Flüssiggels. Liegt es in niedrigviskoser Form vor, so kann es zur Erreichung einer besonders guten Verteilbarkeit auf dem Haar auch versprüht werden. Das erfindungsgemäße Haar behandlungsmittel liegt dann in Kombination mit einer geeigneten mechanisch betriebenen Sprühvorrichtung vor. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann z.B. eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Das erfindungsgemäße Mittel wird angewendet, indem eine für den gewünschten Konditioniereffekt ausreichende Menge in oder auf dem trockenen Haar oder nach der Haarwäsche in oder auf dem nassen oder feuchten Haar verteilt wird. Die anzuwendende Menge hängt von der Haarfülle ab und beträgt typischerweise 1 bis 25 g. Bei der bevorzugten Verwendung als Rinse-Produkt wird nach einer ausreichenden Einwirkzeit von beispielsweise 1 bis 15 Minuten das Haar ausgespült. Anschließend wird das Haar gegebenenfalls durchgekämmt oder zur Frisur geformt und getrocknet. Bei einer Verwendung als Leave-in-Produkt wird das Haar nach Aufbringen des Mittels nicht ausgespült.

Die nachfolgenden Beispiele sollen den Gegenstand der Erfindung näher erläutern.

### Beispiel 1: Klare Haarkur für strapaziertes Haar

| | |
|---|---|
| 3,0 g | Arquad® 12-25 (25%ig, Lauryltrimoniumchlorid) |
| 2,0 g | Abil® 9950 (30%ig, Dimethicone Propyl PG-Betaine) |
| 1,1 g | Pure Thix® HH (Polyether-1, 20%ig in Butylenglykol/Wasser) |
| 0,5 g | Brij® 30 (Laureth-4) |
| ad 100 g | Wasser |

### Beispiel 2: Klare Haarspülung für dauergewelltes Haar

| | |
|---|---|
| 1,0 g | Arquad® 12-50 (50%ig, Lauryltrimoniumchlorid) |
| 0,6 g | Tegobetain® (30%ig in Wasser, Cocamidopropyl Betaine) |
| 0,8 g | Abil® Quat 3272 (50%ig in Propylenglykol, Quaternium-80, diquatemäres Silikon) |
| 0,8 g | Pure Thix® L (PEG-180/Octoxynol-40/TMMG Copolymer) |
| 0,2 g | Glyoxylsäure |
| ad 100 g | Wasser |

### Beispiel 3: Klares Leave-in-Treatment

| | |
|---|---|
| 1,0 g | Tallowtrimoniumchlorid |
| 2,0 g | Abil® S 201 (30%ig in Isopropanol/Wasser, Dimethicone/Sodium PG-Propyldimethicone Thiosulfate Copolymer) |
| 1,1 g | Pure Thix® M (PEG-180/Laureth-50/TMMG Copolymer) |
| 3,0 g | Luviquat® FC 905 (40%ig in Wasser, Polyquatemium-16) |
| ad 100 g | Wasser |

### Beispiel 4: Klare Haarkur zum Entwirren der Haare

| | |
|---|---|
| 2,5 g | Arquad® 12-50 (50%ig, Lauryltrimoniumchlorid) |
| 1,8 g | Abil® Quat 3270 (50%ig in Propylenglykol, Quaternium-80, diquaternäres Silikon) |
| 1,3 g | Pure Thix® HH (20%ig in Butylenglykol/Wasser) |
| 0,3 g | Brij® 30 (Laureth-4) |
| 0,2 g | Zitronensäure |
| ad 100 g | Wasser |

### Beispiel 5: Klare Haarspülung - besonders mild

| | |
|---|---|
| 0,8 g | Arquad® 12-25 (25%ig, Lauryltrimoniumchlorid) |
| 1,0 g | Dow Corning® 193 (Dimethicone Copolyol) |
| 1,0 g | Pure Thix® M (PEG-180/Laureth-50/TMMG Copolymer) |
| 0,5 g | Rewoteric® AM CAS (50%ig in Wasser, Cocamidopropyl Hydroxysultaine) |
| ad 100 g | Wasser |

### Beispiel 6: Klares Leave-in-Treatment

| | |
|---|---|
| 1,0 g | Arquad® 12-50 (50%ig, Lauryltrimoniumchlorid) |
| 2,0 g | Abil® 8863 (Dimethicone Copolyol) |
| 0,9 g | Pure Thix® HH (20%ig in Butylenglykol/Wasser) |
| 0,5 g | Luviskol® K30 (Polyvinylpyyrolidon) |
| ad 100 g | Wasser |

## Patentansprüche

1. Haarbehandlungsmittel mit einem Gehalt an
(A) mindestens einer haarpflegenden Silikonverbindung, welche mindestens eine hydrophile Gruppe enthält und
(B) mindestens einem nichtionischen, amphiphilen Assoziativverdicker, ausgewählt aus hydrophob modifizierten Aminoplast/Polyether Copolymeren
in einer geeigneten kosmetischen Basis.

2. Haarbehandlungsmittel nach Anspruch 1, **dadurch gekennzeichnet, daß** es zusätzlich mindestens einen haarpflegenden Stoff (C) enthält, welcher mindestens eine kationaktive Gruppe aufweist.

3. Mittel nach Anspruch 2, **dadurch gekennzeichnet, daß**der haarpflegende kationaktive Stoff ausgewählt ist aus kationaktiven Tensiden, Polymeren mit kationischen oder kationisierbaren Gruppen, kationisch derivatisierten Proteinen, kationisch derivatisierten Proteinhydrolysaten und Betain.

4. Mittel nach Anspruch 3, **dadurch gekennzeichnet, daß** das kationische Tensid ausgewählt ist aus Verbindungen der allgemeinen Formel (I)
N⁽⁺⁾R¹R²R³R⁴ X⁽⁻⁾ (I)
wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten und X⁽⁻⁾ ein Anion darstellt.

5. Mittel nach Anspruch 3, **dadurch gekennzeichnet, daß** das Polymer mit kationischen oder kationisierbaren Gruppen ausgewählt ist aus Methylvinylimidazoliumchlorid/Vinylpyrrolidon Copolymeren, quatemisierten Vinylpyrrolidon/Dimethylaminoethylmethacrylat Copolymeren, kationisch derivatisierten Polysacchariden, Chitosan, Chitosansalzen und Chitosanderivaten.

6. Mittel nach einem der vorhergehenden Ansprüche, **dadurch gekennzeichnet, daß** die hydrophile Gruppe der Silikonverbindung ausgewählt ist aus Hydroxylgruppen, primären, sekundären oder tertiären Aminogruppen, quaternären Ammoniumgruppen, Alkylenoxidgruppen, betainischen Gruppen und Thiosulfatgruppen.

7. Mittel nach einem der vorhergehenden Ansprüche, **dadurch gekennzeichnet, daß** die Silikonverbindung mit mindestens einer hydrophilen Gruppe ausgewählt ist aus Silikonverbindungen mit kationischen Gruppen, hydroxysubstituierten Siloxanen, Siloxan/Polyoxyalkylen Copolymeren und aminosubstituierten Siloxanen.

8. Mittel nach einem der vorhergehenden Ansprüche, **dadurch gekennzeichnet, daß** der Verdicker ausgewählt ist aus Polymeren der allgemeinen Formel (VI) 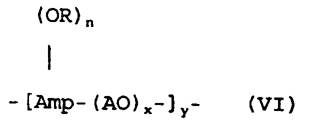 wobei Amp ein Aminoplastmonomer oder den Rest eines Aminoplastoligomers oder -polymers bedeutet, AO für eine Alkylenoxidgruppe steht, R für Wasserstoff, C1-C4-Alkyl oder C1-C4-Acyl steht und x und y Zahlen größer 1 sind.

9. Mittel nach einem der vorhergehenden Ansprüchen, **dadurch gekennzeichnet, daß** der Verdicker ausgewählt ist aus den Reaktionsprodukten der säurekatalysierten Reaktion von Glycolurilderivaten und Polyalkylenglykolen und alkoxylierten Kohlenwasserstoffen.

10. Mittel nach einem der vorhergehenden Ansprüchen, **dadurch gekennzeichnet, daß** der Verdicker ausgewählt ist aus Polyether-1, PEG-180/Octoxynol-40/TMMG Copolymer und PEG-180/Laureth-50/TMMG Copolymer.

11. Mittel nach einem der vorhergehenden Ansprüche, **dadurch gekennzeichnet, daß** es in einer optisch klaren Form vorliegt.

12. Mittel nach einem der vorhergehenden Ansprüche, **dadurch gekennzeichnet, daß** es in einer durchsichtigen oder durchscheinenden Verpackung vorliegt.

## Claims

1. Hair-treatment agent with a content of
(A) at least one haircare silicone compound which contains at least one hydrophilic group and
(B) at least one nohionic, amphiphilic associative thickener chosen from hydrophobically modified aminoplast/polyether copolymers
in a suitable cosmetic base.

2. Hair-treatment agent according to Claim 1, **characterized in that** it additionally comprises at least one haircare substance (C) which has at least one cation-active group.

3. Agent according to Claim 2, **characterized in that** the haircare cation-active substance is chosen from cation-active surfactants, polymers with cationic or cationizable groups, cationically derivatized proteins, cationically derivatized protein hydrolysates and betaine.

4. Agent according to Claim 3, **characterized in that** the cationic surfactant is chosen from compounds of the general formula (I)
N⁽⁺⁾R¹R²R³R⁴ X⁽⁻⁾ (I) (I)
where R1 to R4, independently of one another, are aliphatic groups, aromatic groups, alkoxy groups, polyoxyalkylene groups, alkylamido groups, hydroxyalkyl groups, aryl groups or alkaryl groups having 1 to 22 carbon atoms and X⁽⁻⁾ is an anion.

5. Agent according to Claim 3, **characterized in that** the polymer with cationic or cationizable groups is chosen from methylvinylimidazolium chloride/vinylpyrrolidone copolymers, quaternized vinylpyrrolidone/dimethylaminoethyl methacrylate copolymers, cationically derivatized polysaccharides, chitosan, chitosan salts and chitosan derivatives.

6. Agent according to one of the preceding claims, **characterized in that** the hydrophilic group of the silicone compound is chosen from hydroxyl groups, primary, secondary or tertiary amino groups, quaternary ammonium groups, alkylene oxide groups, betainic groups and thiosulphate groups.

7. Agent according to one of the preceding claims, **characterized in that** the silicone compound with at least one hydrophilic group is chosen from silicone compounds with cationic groups, hydroxy-substituted siloxanes, siloxane/polyoxyalkylene copolymers and amino-substituted siloxanes.

8. Agent according to one of the preceding claims, **characterized in that** the thickener is chosen from polymers of the general formula (VI) 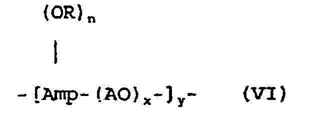 where Amp is an aminoplast monomer or the radical of an aminoplast oligomer or polymer, AO is an alkylene oxide group, R is hydrogen, C1-C4-alkyl or C1-C4-acyl and x and y are numbers greater than 1.

9. Agent according to one of the preceding claims, **characterized in that** the thickener is chosen from the reaction products of the acid-catalysed reaction of glycoluril derivatives and polyalkylene glycols and alkoxylated hydrocarbons.

10. Agent according to one of the preceding claims, **characterized in that** the thickener is chosen from polyether-1, PEG-180/octoxynol-40/TMMG copolymer and PEG-180/laureth-50/TMMG copolymer.

11. Agent according to one of the preceding claims, **characterized in that** it is in an optically clear form.

12. Agent according to one of the preceding claims, **characterized in that** it is in a transparent or translucent packaging.

## Revendications

1. Composition de traitement capillaire ayant une teneur en
(A) au moins un composé silicone de soin capillaire, qui comporte au moins un groupe hydrophile et
(B) au moins un épaississant associatif amphiphile non ionique choisi parmi des copolymères aminoplaste/polyéther à modification hydrophobe,
dans une base cosmétique appropriée.

2. Composition de traitement capillaire selon la revendication 1, **caractérisée en ce qu'**elle contient au moins une substance de soin capillaire (C) qui comporte au moins un groupe cationique.

3. Composition selon la revendication 2, **caractérisée en ce que** la substance cationique de soin capillaire est choisie parmi des tensioactifs cationiques, des polymères à groupes cationiques ou pouvant être convertis en cations, des protéines à fonctionnalisation cationique, des hydrolysats de protéines à fonctionnalisation cationique et la bétaïne.

4. Composition selon la revendication 3, **caractérisée en ce que** le tensioactif cationique est choisi parmi des composés de formule générale (I)
N⁽⁺⁾R¹R²R³R⁴ X⁽⁻⁾ (I)
dans laquelle R¹ à R⁴ représentent, indépendamment les uns des autres, des groupes aliphatiques, des groupes aromatiques, des groupes alcoxy, des groupes polyoxyalkylène, des groupes alkylamido, des groupes hydroxyalkyle, des groupes aryle ou alkaryle ayant de 1 à 22 atomes de carbone, et X⁽⁻⁾ représente un anion.

5. Composition selon la revendication 3, **caractérisée en ce que** le polymère à groupes cationiques ou pouvant être convertis en cations est choisi parmi des copolymères chlorure de méthylvinylimidazolium/vinylpyrrolidone, des copolymères vinylpyrrolidone/méthacrylate de diméthylaminoéthyle rendus quaternaires, des polysaccharides à fonctionnalisation cationique, le chitosane, des sels de chitosane et des dérivés de chitosane.

6. Composition selon l'une quelconque des revendications précédentes, **caractérisée en ce que** le groupe hydrophile du composé silicone est choisi parmi des groupes hydroxy, des groupes amino primaires, secondaires ou tertiaires, des groupes ammonium quaternaire, des groupes oxyde d'alkylène, des groupes de type bétaïne et des groupes thiosulfate.

7. Composition selon l'une quelconque des revendications précédentes, **caractérisée en ce que** le composé silicone comportant au moins un groupe hydrophile est choisi parmi des composés silicone à groupes cationiques, des siloxanes substitués par hydroxy, des copolymères siloxane/polyoxyalkylène et des siloxanes substitués par amino.

8. Composition selon l'une quelconque des revendications précédentes, **caractérisée en ce que** l'épaississant est choisi parmi des polymères de formule générale (VI) 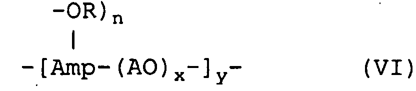 dans laquelle Amp représente un monomère aminoplaste ou le reste d'un oligomère ou polymère aminoplaste, AO représente un groupe oxyde d'alkylène, R représente un atome d'hydrogène, un groupe alkyle en C₁-C₄ ou acyle en C₁-C₄, x et y sont des nombres supérieurs à 1.

9. Composition selon l'une quelconque des revendications précédentes, **caractérisée en ce que** l'épaississant est choisi parmi les produits de réaction de la réaction, catalysée par un acide, de dérivés de glycolurile et de polyalkylèneglycols et d'hydrocarbures alcoxylés.

10. Composition selon l'une quelconque des revendications précédentes, **caractérisée en ce que** l'épaississant est choisi parmi le polyéther-1, le copolymère PEG-180/octoxynol-40/TMMG et le copolymère PEG-180/laureth-50/TMMG,

11. Composition selon l'une quelconque des revendications précédentes, **caractérisée en ce qu'**elle se trouve sous une forme optiquement limpide.

12. Composition selon l'une quelconque des revendications précédentes, **caractérisée en ce qu'**elle est présente dans un conditionnement transparent ou translucide.
